# EUROPÄISCHE PATENTSCHRIFT

(11) **EP 3 351 667 B1**
(45) Veröffentlichungstag und Bekanntmachung des Hinweises auf die Patenterteilung: **30.10.2019**
(21) Anmeldenummer: 17188472.9
(22) Anmeldetag: 30.08.2017
(51) Int. Cl.: D04B 1/10, D04B 1/24, A61F 13/08

(54) **KOMPRESSIONS-LEIBTEIL ODER KOMPRESSIONSSTRUMPFHOSE MIT LEIBTEIL ZUR KOMPRESSIONSBEHANDLUNG WÄHREND DER SCHWANGERSCHAFT**
COMPRESSION BELT OR COMPRESSION STOCKING WITH BELT FOR COMPRESSION TREATMENT DURING PREGNANCY
CORSAGE DE CONTENTION OU COLLANT DE CONTENTION POURVU DE CORSAGE PERMETTANT LE TRAITEMENT PAR COMPRESSION PENDANT LA GROSSESSE

(30) Priorität: 20.01.2017 DE 202017100286 U
(43) Veröffentlichungstag der Anmeldung: 25.07.2018
(73) Patentinhaber: Julius Zorn GmbH, 86551 Aichach (DE)
(72) Erfinder: Lechner, Siegfried, 86529 Schrobenhausen (DE)
(74) Vertreter: Charrier Rapp & Liebau

(56) Entgegenhaltungen:
- DE-A1-102011 000 624
- DE-C- 712 909
- DE-U- 7 032 243
- FR-A1- 2 936 409
- JP-A- 2007 169 820
- JP-U- S57 205 206
- KR-B1- 101 540 906
- US-A1- 2001 054 303
- US-A1- 2003 019 252

## Beschreibung

Die Erfindung betrifft ein Kompressions-Leibteil oder Kompressionsstrumpfhose mit Leibteil zur Kompressionsbehandlung, insbesondere von Lip- oder Lymphödemen oder einer Varikosis, während der Schwangerschaft, gemäß dem Oberbegriff des Anspruchs 1.

Für die Kompressionsbehandlung von Lipödemen oder Lymphödemen sind flach- oder rundgestrickte Kompressionsartikel bekannt. Die EP 2 113 593 A1 offenbart beispielsweise ein Rundgestrick für die Kompressionstherapie von Lymphödemen. Aus solchen flach- oder rundgestrickten Kompressionsgestricken können beispielsweise Kompressionsstrümpfe oder Ärmel hergestellt werden, welche beim Anlegen an eine Körperextremität, beispielsweise einem Bein oder einem Arm, einen Kompressionsdruck auf die Körperextremität ausüben. Durch den Kompressionsdruck wird das Gewebe der Körperextremität komprimiert und der Abfluss von Lymphflüssigkeit sowie die Resorption von Ödemen gefördert.

Bekannte Kompressionsgestricke, die in der Kompressionstherapie von Lip- und Lymphödemen eingesetzt werden können, sind aus einem Grundgestrick mit einem darin eingelegten oder eingebundenen elastischen Schussfaden gebildet. Das Grundgestrick umfasst in der Regel einen maschenbildend verstrickten Gestrickfaden aus einem textilen Material mit keiner oder geringer Elastizität und ggf. einen weiteren, maschenbildend verstrickten elastischen Faden. Durch den ggf. maschenbildend eingestrickten elastischen Faden wird das Grundgestrick in Längsrichtung elastisch. Die Elastizität des Kompressionsgestricks in Querrichtung wird durch den elastischen Schussfaden bewirkt, der die Kompression auf die Körperextremität bewirkt, wenn das Kompressionsgestrick beispielsweise in Form eines Kompressionsstrumpfs oder -ärmels an einer Körperextremität, wie einem Bein oder einem Arm, angelegt wird.

Während der Schwangerschaft ist das Tragen eines Kompressionstextils, wie beispielsweise einer Kompressionsstrumpfhose oder eines Kompressions-Leibteils, im Bauchbereich wegen des vom Kompressionsgestrick ausgeübten Kompressionsdrucks problematisch. Zum Einen kann das Anlegen eines Kompressionsgestricks im Bauchbereich während der Schwangerschaft durch den hohen Kompressionsdruck zu einer Beeinträchtigung der Gebärmutter und des Fötus führen. Zum Anderen wird die Kompressionswirkung des Kompressionstextils aufgrund des sich während der Schwangerschaft ändernden Bauchumfangs beeinflusst, da der vom Kompressionstextil ausgeübte Kompressionsdruck abhängig vom Körperumfang ist. Es kann daher während der Schwangerschaft kein gleichbleibender Kompressionsdruck sicher gestellt werden.

Um in jeder Phase der Schwangerschaft eine einwandfreie Passform und einen abgestimmten Stütz- und Straffungs-Effekt zu erzielen, wird in der DE 70 061 72 U1 eine Umstands-Stützstrumpfhose vorgeschlagen, welche einen Miederslip umfasst, der sowohl im Rückteil wie auch im Vorderteil einen Zwickel aufweist und dessen Bund verstellbar ausgearbeitet ist.

Aus der DE 70 149 15 U1 ist eine medizinische Strumpfhose, insbesondere eine Gummistrumpfhose, bekannt, welche einen Einschnitt aufweist, der sich bei Verwendung während der Schwangerschaft zu einem Ausschnitt erweitert. Dadurch kann die medizinische Strumpfhose in eine Umstandshose umgewandelt und während der gesamten Schwangerschaft getragen werden. Die Größe des Ausschnitts kann dabei durch Bänder, beispielsweise durch ein weichelastisches Gummiband, das in einem Saum an der oberen Kante der Strumpfhose geführt ist, geregelt werden.

Die KR 101 540 906 B1 zeigt eine Umstandsstrumpfhose deren Bund an den aktuellen Bauchumfang angepasst werden kann.

Mit den bekannten Umstands-Strumpfhosen können die eingangs geschilderten Probleme beim Tragen eines Kompressions-Leibteils während der Schwangerschaft jedoch nur zum Teil gelöst werden. Insbesondere wird auf den Bauch ein Kompressionsdruck ausgeübt, der die Gebärmutter und den Fötus beeinträchtigen kann und außerhalb des Bauchbereichs ist kein gleichförmiger und während jeder Schwangerschaftsphase gleichbleibender Kompressionsdruck gewährleistet.

Hiervon ausgehend liegt der Erfindung die Aufgabe zugrunde, ein Kompressions-Leibteil oder eine Kompressionsstrumpfhose mit einem Leibteil zur Kompressionsbehandlung während der Schwangerschaft bereitzustellen, die in jeder Phase der Schwangerschaft außerhalb des Bauchbereichs der Patientin einen gleichbleibenden und gleichförmigen Kompressionsdruck auf das darunter liegende Körpergewebe ausübt, ohne die Gebärmutter oder den Fötus zu beeinträchtigen. Das Kompressions-Leibteil oder die Kompressionsstrumpfhose mit Leibteil soll dabei möglichst einfach anziehbar sein und eine zumindest weitgehend selbsttätige Anpassung des Leibteils an den sich während der Schwangerschaft ändernden Körperumfang ermöglichen.

Diese Aufgaben werden mit einem Kompressions-Leibteil oder einer Kompressionsstrumpfhose mit einem Leibteil gemäß Anspruch 1 gelöst. Bevorzugte Ausführungsformen des erfindungsgemäßen Kompressions-Leibteils bzw. der erfindungsgemäßen Kompressionsstrumpfhose mit Leibteil können den abhängigen Ansprüchen entnommen werden.

Gemäß der Erfindung umfasst das Leibteil einen posterioren Kompressionsabschnitt aus einem Kompressionsgestrick mit einem kompressionsgebenden Schussfaden, sowie einen anterioren Bauchabschnitt, der bei angelegtem Leibteil im Bauchbereich der Patienten zu liegen kommt, wobei der Bauchabschnitt erfindungsgemäß aus einem dehnbaren Gestrick ohne Schussfaden und mit einer Mehrzahl von Öffnungen im Gestrick gebildet ist, wobei die Öffnungen in dem Grundgestrick des Bauchabschnitts netzartig, gitterartig oder perforationsartig ausgebildet sind. Die Öffnungen im Gestrick des Bauchabschnitts bilden dabei bevorzugt ein regelmäßiges, matrixförmiges Lochmuster nach Art einer Perforation oder eines Netzes. Durch dieses Lochmuster wird das Gestrick des Bauchabschnitts sowohl in Längsrichtung als auch in Umfangsrichtung des Leibteiles besser dehnbar und ermöglicht dadurch eine selbsttätige Anpassung an den sich während der Schwangerschaft ändernden Bauchumfang der Patientin. Weiterhin erhöht das Lochmuster die Atmungsaktivität im Bauchabschnitt. Unter "Öffnungen" werden dabei nicht die offenen Bereich in dem Gestrick des Bauchabschnitts verstanden, die bei jedem Gestrick vorhanden sind, sondern zusätzliche Ausnehmungen bzw. Durchbrüche in dem Gestrick des Bauchabschnitt, die darin insbesondere als regelmäßiges Lochmuster angeordnet sind und dadurch ein Gitter- bzw. Netzmuster ausbilden.

Das insbesondere durch die Öffnungen dehnbare Gestrick des Bauchabschnitts übt dabei wegen des im Bauchabschnitt nicht vorhandenen Schussfadens keinen Kompressionsdruck auf den Bauchabschnitt aus. Die Öffnungen in dem Gestrick des Bauchabschnitts ermöglichen jedoch eine Dehnung des Leibteils im Bauchabschnitt und damit eine automatische Anpassung an den sich während der Schwangerschaft ändernden Körperumfang der Patientin im Bauchbereich. Gleichzeitig wird durch den posterioren Kompressionsabschnitt die Ausübung eines vom Kompressionsgestrick des Kompressionsabschnitts erzeugten Kompressionsdrucks auf das darunter liegende Gewebe außerhalb des Bauchbereichs gewährleistet, so dass das Leibteil außerhalb des Bauchbereichs, insbesondere im Gesäß- und Lendenbereich der Patientin, durch die Ausübung eines Kompressionsdrucks eine Verbesserung des Abflusses von Lymphflüssigkeit bewirkt und eine Resorption von Lip- oder Lymphödemen unterstützt. Ferner erhöht sich der Tragekomfort, da durch die Öffnungen im Bauchabschnitt die Atmungsaktivität des Leibteils erhöht wird.

In einem bevorzugten Ausführungsbeispiel der Erfindung ist der Bauchabschnitt des Leibteils aus einem Rechts-Rechts-Gestrick ohne Schussfaden gebildet, wobei in dem Gestrick Öffnungen eingestrickt sind, die ein regelmäßiges, matrixartiges Lochmuster in dem Gestrick des Bauchabschnitts erzeugen. Die Öffnungen in dem Gestrick des Bauchabschnitts können dabei beispielsweise wie bei einem Petinet-Muster in das Grundgestrick durch Abnahme von Maschen auf ihrer Nadel und Umhängen auf ihre Nachbarnadel eingestrickt werden.

Zweckmäßig ist der Bauchabschnitt durch ein elastisches Flachgestrick aus einem elastischen Strickfaden gebildet. Das Gestrick des Bauchabschnitts kann durch eine Naht mit dem sich randseitig an den Bauchabschnitt anschließenden posterioren Kompressionsabschnitt verbunden sein.

Das Gestrick des Bauchabschnitts ist jedoch bevorzugt zur Verbesserung des Tragekomforts nahtlos mit dem Kompressionsgestrick des sich randseitig an den Bauchabschnitt anschließenden posterioren Kompressionsabschnitt verbunden. Eine nahtlose Verbindung kann bspw. erzeugt werden, indem das Gestrick des Bauchabschnitts mit dem Kompressionsgestrick verstrickt wird. Dabei kann eine einfache Herstellung des Leibteils durch Stricken gewährleistet werden, wenn das Grundgestrick des Kompressionsgestricks des posterioren Kompressionsabschnitts nach seiner Strickart und dem verwendeten Strickfaden dem Grundgestrick des Bauchabschnitts entspricht und in einem Strickvorgang mit dem Gestrick des Bauchabschnitts verstrickt wird, wobei nur in dem posterioren Kompressionsabschnitt ein elastischer und kompressionsgebener Schussfaden in das Grundgestrick eingelegt oder darin eingebunden wird und ggf. noch ein weiterer Strickfaden maschenbildend eingestrickt wird.

Zweckmäßig erzeugt das Kompressionsgestrick des posterioren Kompressionsabschnitts bei angelegtem Leibteil auf das darunter liegende Körpergewebe einen von proximal nach distal zunehmenden Kompressionsdruck, insbesondere im Gesäß- und Lendenbereich der Patientin. Wenn sich an das Leibteil Beinteile anschließen, um eine Kompressionshose oder eine Kompressionsstrumpfhose mit einem Fußteil auszubilden, ist es zweckmäßig, wenn im gesamten Bereich der Kompressionshose bzw. der Kompressionsstrumpfhose ein von proximal nach distal zunehmender Kompressionsdruck auf die Beine sowie den Gesäß- und Lendenbereich der Patientin ausgeübt wird. Die Ausbildung von Kompressionsgestricken mit einem derartigen Kompressionsgradienten ist aus dem Stand der Technik der Kompressionsstrümpfe bekannt. Wenn der Kompressionsabschnitt einen entsprechenden Kompressiongradienten aufweist, wird bei angelegter Kompressionshose bzw. Kompressionsstrumpfhose der venöse Blutfluß unterstützt und beschleunigt. Dadurch können während der Schwangerschaft Varikosen therapiert oder vorgebeugt werden. Insbesondere die während einer Schwangerschaft entstehenden und als Schwangerschaftsvarikosis bekannten Varizen sowie Schwangerschaftsödeme können so therapiert oder sogar ganz unterbunden werden. Zweckmäßig erzeugt das Kompressionsgestrick des anterioren Kompressionsabschnitts dabei einen Kompressionsdruck auf das darunter liegende Körpergewebe der Patientin aus, der im Bereich einer der Kompressionsklassen I bis IV liegt, die in der DIN 58133 bzw. dem RAL-Gütezeichen RAL-GZ 387/1 definiert sind.

Um außerhalb des Bauchbereichs einen gleichförmigen Kompressionsdruck auszuüben, ist es vorteilhaft, wenn sich im anterioren Bereich des Leibteils distal des Bauchabschnitts ein anteriorer Kompressionsabschnitt aus einem Kompressionsgestrick anschließt, wobei das Kompressionsgestrick dieses anterioren Kompressionsabschnitts zweckmäßig identisch zu dem Kompressionsgestrick des posterioren Kompressionsabschnitts (auf gleicher Höhe des Leibteils) ausgebildet ist. Zweckmäßig schließt sich der anteriore Kompressionsabschnitt in Umfangsrichtung nahtlos und aus demselben Kompressionsgestrick an den posterioren Kompressionsabschnitt an. Da auch der Bauchabschnitt bevorzugt nahtlos in dem Leibteil eingearbeitet, insbesondere eingestrickt ist, kann somit insgesamt ein Kompressionsteil erzeugt werden, das ohne Nähte auskommt, wodurch der Tragekomfort verbessert wird.

Der posteriore Kompressionsabschnitt und entsprechend auch der gegebenenfalls vorhandene anteriore Kompressionsabschnitt können beispielsweise aus einem Rechts-Rechts-Flachgestrick mit darin eingebundenem oder eingelegtem Schussfaden gebildet sein, wobei der kompressionsgebende Schussfaden des Kompressionsgestricks sich in Umfangsrichtung erstreckt.

Zur zusätzlichen Anpassung des Umfangs des erfindungsgemäßen Leibteils an dessen proximalen Ende (im proximalen Bauch und Lendenbereich) ist zweckmäßig an einem proximalen Randabschnitt des Leibteils ein verstellbarer Taillengurt angeordnet, der ein verstellbares Verschlusselement, beispielsweise einen Klettverschluss oder einen Verschlussknopf mit einem zugehörigen Knopflochband mit mehreren Knopflöchern, aufweist. Durch das verstellbare Verschlusselement kann der Umfang des Leibteils im Taillenbereich der Patientin an den sich während der Schwangerschaft ändernden Körperumfang angepasst werden. Dies ermöglicht ein einfaches Anlegen des Leibteils, verhindert beim Tragen ein Verrutschen und erhöht den Tragekomfort.

An das Kompressionsleibteil gemäß der Erfindung können zwei Beinteile angebracht werden, insbesondere durch Annähen oder Anstricken. Die Beinteile können dabei unterschiedliche Längen aufweisen, um eine Hose in Leggings-, Capri- oder Bermuda-Form zu erzeugen. Die Hose kann auch an beiden Beinteilen einen nahtlos angeformten, insbesondere angstrickten Fußteil umfassen und somit eine Kompressionsstrumpfhose ausbilden.

Diese und weitere Vorteile des Kompressions-Leibteils bzw. einer erfindungsgemäßen Kompressionsstrumpfhose mit Leibteil gemäß der Erfindung ergeben sich aus dem nachfolgend unter Bezugnahme auf die begleitenden Zeichnungen näher beschriebenen Ausführungsbeispiel, wobei die Zeichnungen zeigen:
- **Figur 1:**: Vorderansicht eines an den Unterkörper einer Patientin angelegten Kompressionsleibteils gemäß der Erfindung;
- **Figur 2:**: Seitenansicht des Kompressions-Leibteils von Figur 1;
- **Figur 3:**: Strickbild des Gestricks des Bauchabschnitts des Leibteils von Figur 1.

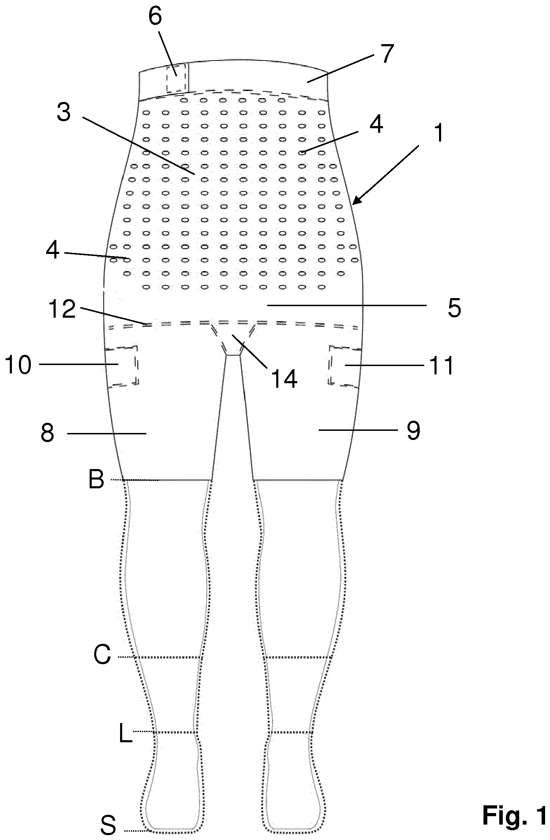

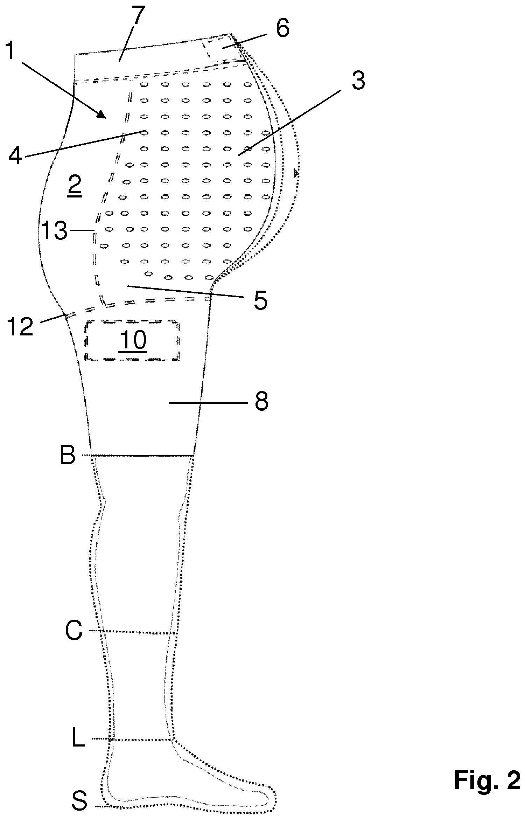

In den Figuren 1 und 2 ist ein Kompressions-Leibteil 1 gemäß der Erfindung dargestellt, wobei das Leibteil 1 am Unterkörper einer schwangeren Patientin angelegt ist. In der Seitenansicht der Figur 2 ist dabei der sich während der Schwangerschaft ändernde Bauchumfang der Patientin in gestrichelten Linien sowie mit einer nach außen weisenden Pfeilspitze angedeutet.

Das Leibteil 1 umfasst dabei einen posterioren Kompressionsabschnitt 2, der bei angelegtem Leibteil im Gesäß- und Lendenbereich der Patientin zu liegen kommt (wie aus Figur 2 ersichtlich). Das Leibteil 1 umfasst weiterhin einen anterioren Bauchabschnitt 3, der bei angelegtem Leibteil im Bauchbereich der Patientin zu liegen kommt. Der posteriore Kompressionsabschnitt 2 und der anteriore Bauchabschnitt 3 können dabei, wie in Figur 2 gezeigt, durch eine Naht 13 miteinander vernäht sein. Zur Verbesserung des Tragekomforts ist es jedoch vorteilhaft, wenn der posteriore Kompressionsabschnitt 2 und der anteriore Bauchabschnitt 3 nahtlos miteinander verbunden und insbesondere miteinander verstrickt sind.

Der posteriore Kompressionsabschnitt 2 ist aus einem Kompressionsgestrick gebildet, welches zumindest ein Grundgestrick aus einem unelastischen oder zumindest im Wesentlichen unelastischen Gestrickfaden und einen darin eingelegten oder eingebundenen elastischen Schussfaden aufweist. Der elastische Schussfaden erzeugt dabei eine Kompressionswirkung auf das darunter liegende Körpergewebe, wenn das Kompressionsgestrick an ein Körperteil angelegt ist. Das Kompressionsgestrick des Kompressionsabschnitts 2 kann neben dem unelastischen Strickfaden zusätzlich einen elastischen Strickfaden enthalten, der zusammen mit dem unelastischen Strickfaden zur Ausbildung eines Grundgestricks verstrickt wird. Bei dem Kompressionsgestrick handelt es sich in diesem Fall dann um ein sowohl in Längs- als auch in Querrichtung dehnbares Kompressionsgestrick (sogenannte Zweizug-Ware). Die Kompressionswirkung des Kompressionsgestricks wird dabei in erster Linie durch die Elastizität des in Querrichtung verlaufenden elastischen Schussfadens hervorgerufen. Zur Herstellung des Kompressionsgestricks können Polyamid-, Elasthan-, Baumwoll-, Elastodien- und Viskose-Fäden oder Microfasern, auch in Kombination miteinander, insbesondere auch in Form von umwundenen bzw. umwickelten Kernfäden eingesetzt werden.

Der anteriore Bauchabschnitt 3 kann beispielsweise aus einem Rechts-Rechts-Gestrick gestrickt sein und weist keinen Schussfaden auf. Durch das Fehlen eines Schussfadens in dem Gestrick des Bauchabschnitts 3 erzeugt das Leibteil 1 im Bauchabschnitt keinen Kompressionsdruck auf das darunter liegende Körpergewebe.

Das gesamte Leibteil 1 mit dem Bauchabschnitt 3 und dem posterioren Kompressionsabschnitt 2 ist bevorzugt als einlagiges Gestrick und ohne Nähte ausgebildet und weist insbesondere kein Futter oder ähnliche Abdeckungen auf dem Gestrick auf.

Wie aus den Figuren 1 und 2 ersichtlich, weist das Gestrick des anterioren Bauchabschnitts 3 netzartige bzw. perforationsartige Öffnungen 4 auf. Die Öffnungen 4 sind dabei zweckmäßig gleichartig und in regelmäßiger Anordnung in dem Gestrick des Bauchabschnitts 3 verteilt und bilden insbesondere ein regelmäßiges und matrixartiges Lochmuster in dem Gestrick aus.

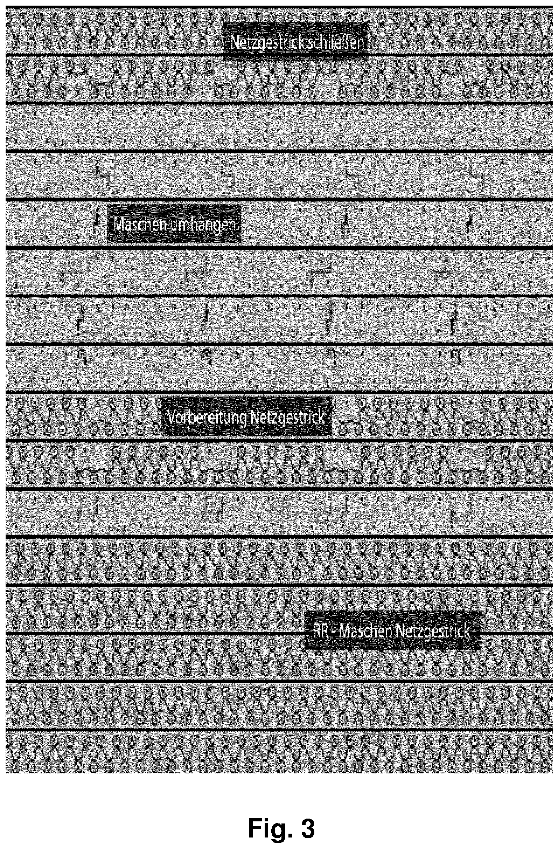

Diese Öffnungen 4 sind beispielsweise nach Art eines Petinet-Musters in das Grundgestrick des Bauchabschnitts 3 eingestrickt. Das Einstricken der Öffnungen 4 kann beispielsweise beim Verstricken eines Strickfadens zu dem Gestrick des Bauchabschnitts 3 durch Abnahme von Maschen auf ihrer Nadel und Umhängen auf ihre Nachbarnadel erfolgen, bspw. wie bei einem 2-Nadel-Petinet-Gestrick. Ein Strickbild für das Gestrick des Bauchabschnitts 3 ist beispielhaft in Figur 3 zusammen mit den einzelnen Schritten beim Stricken des Gestricks dargestellt. Das Gestrick des Bauchabschnitts 3 ist in Figur 3 mit "Netzgestrick" gekennzeichnet, da das Gestrick des Bauchabschnitts 3 netzartige Öffnungen 4 aufweist.

Vorteilhaft ist es, wenn das Gestrick des Bauchabschnitts 3 hinsichtlich der Strickart und des verwendeten Strickfadens mit dem Grundgestrick des Kompressionsgestricks des posterioren Kompressionsabschnitts 2 übereinstimmt. In diesem Fall kann der posteriore Kompressionsabschnitt 2 mit den seitlichen Rändern des sich in Umfangsrichtung daran anschließenden anterioren Bauchabschnitts 3 nahtlos verstrickt werden.
Wie aus Figur 1 ersichtlich, schließt sich im anterioren Bereich des Leibteils 1 distal des Bauchabschnitts 3 ein anteriorer Kompressionsabschnitt 5 an. Der anteriore Kompressionsabschnitt 5 ist wie der posteriore Kompressionsabschnitt 2 ebenfalls aus einem Kompressionsgestrick gebildet. Zweckmäßig handelt es sich um dasselbe Kompressionsgestrick, sowohl in Bezug auf die Strickart als auch die verwendeten Strick- und Schussfäden, wie im posteriore Kompressionsabschnitt 2. Bevorzugt geht der posteriore Kompressionsabschnitt 2 auf Höhe des anterioren Kompressionsabschnitts 5 (also im Bereich distal des Bauchabschnitts 3) nahtlos in den anterioren Kompressionsabschnitt 5 unter Beibehaltung desselben Kompressionsgestricks wie im posterioren Kompressionsabschnitt 2 über.

Wie weiterhin aus den Figuren 1 und 2 ersichtlich, schließen sich an das Leibteil 1 zwei Beinteile 8, 9 an. Die Beinteile 8, 9 können dabei entweder durch eine Quernaht 12, die in den Figuren 1 und 2 gestrichelt dargestellt ist, mit dem distalen Rand des Leibteils 1 vernäht sein. Es ist jedoch auch möglich, die Beinteile 8, 9 nahtlos am distalen Rand des Leibteils 1 durch Verstricken anzusetzen. Zweckmäßig enthalten die Beinteile 8 und 9 ein Kompressionsgestrick entsprechend dem Kompressionsgestrick im posterioren und anterioren Kompressionsabschnitt. Bevorzugt erzeugt das zusammengesetzte Kompressionsgestrick der Beinteile 8, 9 sowie des posterioren und anterioren Kompressionsabschnitts 2, 5 einen von distal nach proximal (also von den Beinteilen 8, 9 in Richtung des posterioren Kompressionsabschnitts 2) abnehmenden Kompressionsdruck. Hierfür ist das Kompressionsgestrick im Bereich der Beinteile 8, 9 weniger dehnbar als im Bereich des posterioren Kompressionsabschnitts 2 ausgebildet.

Die Länge der Beinteile 8, 9 ist variabel und kann - wie aus den Figuren 1 und 2 ersichtlich - die Länge einer Bermuda-Hose (B), einer Carpi-Hose (C), einer Leggins (L) oder einer Strumpfhose mit Fußteil (S) aufweisen.

Um ein Verrutschen der Beinteile 8, 9 im Hüftbereich der Patientin zu verhindern, ist es zweckmäßig, wenn an der Innenseite der Beinteile 8, 9, insbesondere im lateralen Bereich, Haftbänder angeordnet sind. Solche Haftbänder sind in den Figuren 1 und 2 gestrichelt angedeutet und mit Bezugszeichen 10 und 11 gekennzeichnet.

Im Übergangsbereich zwischen dem distalen Rand des Leibteils 1 und den Beinteilen 8, 9 ist zwischen den beiden Beinteilen zweckmäßig ein Zwickel 14 angeordnet. Dieser ist am distalen Rand des Leibteils 1 und den medialen Seitenrändern der Beinteile 8, 9 angenäht.

Das Leibteil 1 oder eine mit einem solchen Leibteil ausgestattete Kompressions(strumpf)hose kann während der Schwangerschaft zur Kompressionsbehandlung von Lip- oder Lymphödemen und/oder Varikosen angezogen werden, wobei der elastische Bauchabschnitt 3 mit den darin ausgeformten und zweckmäßig regelmäßig angeordneten Öffnungen 4 eine dehnbare Anpassung des Umfangs des Leibteils 1 an den sich während der Schwangerschaft ändernden Körperumfang der Patientin gewährleistet. Gleichzeitig wird von dem Kompressionsgestrick des posterioren Kompressionsabschnitts 2 und des optional vorhandenen anterioren Kompressionsabschnitts 5 ein Kompressionsdruck auf das Körpergewebe der Patientin außerhalb des Bauchbereichs erzeugt, der eine Kompressionsbehandlung von Ödemen und Varikosen während der Schwangerschaft ermöglicht, ohne dass ein zu hoher Druck auf den Fötus erzeugt wird.

## Patentansprüche

1. Kompressions-Leibteil oder Kompressionsstrumpfhose mit Leibteil zur Kompressionsbehandlung von Ödemen oder einer Varikosis während der Schwangerschaft, wobei das Leibteil (1) einen posterioren Kompressionsabschnitt (2) aus einem Kompressionsgestrick mit einem kompressionsgebenden Schussfaden sowie einen anterioren Bauchabschnitt (3) aufweist, der bei angelegtem Leibteil (1) im Bauchbereich der Patientin zu liegen kommt, **dadurch gekennzeichnet, dass** der Bauchabschnitt (3) aus einem dehnbaren Gestrick ohne Schussfaden mit einer Mehrzahl von Öffnungen (4) gebildet ist, wobei die Öffnungen (4) in dem Grundgestrick des Bauchabschnitts (3) netzartig, gitterartig oder perforationsartig ausgebildet sind.

2. Kompressions-Leibteil oder Kompressionsstrumpfhose nach Anspruch 1, **dadurch gekennzeichnet, dass** der Bauchabschnitt (3) aus einem Rechts-Rechts-Gestrick ohne Schussfaden mit gitter- oder netzartigen Öffnungen (4) gebildet ist.

3. Kompressions-Leibteil oder Kompressionsstrumpfhose nach Anspruch 1 oder 2, **dadurch gekennzeichnet, dass** der Bauchabschnitt (3) aus einem Gestrick ohne Schussfaden mit einem Petinet-Lochmuster gebildet ist.

4. Kompressions-Leibteil oder Kompressionsstrumpfhose nach einem der voranstehenden Ansprüche, **dadurch gekennzeichnet, dass** der Bauchabschnitt (3) ein elastisches Flachgestrick aus einem elastischen Strickfaden enthält.

5. Kompressions-Leibteil oder Kompressionsstrumpfhose nach einem der voranstehenden Ansprüche, **dadurch gekennzeichnet, dass** das Gestrick des Bauchabschnitts (3) mit dem Kompressionsgestrick des sich randseitig an den Bauchabschnitt (3) anschließenden posterioren Kompressionsabschnitts (2) verstrickt ist.

6. Kompressions-Leibteil oder Kompressionsstrumpfhose nach einem der voranstehenden Ansprüche, **dadurch gekennzeichnet, dass** die Öffnungen (4) in dem Gestrick des Bauchabschnitts (3) durch Abnahme von Maschen auf ihrer Nadel und Umhängen auf ihre Nachbarnadel eingestrickt sind.

7. Kompressions-Leibteil oder Kompressionsstrumpfhose nach einem der voranstehenden Ansprüche, **dadurch gekennzeichnet, dass** das Kompressionsgestrick des posterioren Kompressionsabschnitts (2) bei angelegtem Leibteil (1) einen von proximal nach distal zunehmenden Kompressionsdruck auf den Gesäß- und Lendenbereich der Patientin erzeugt.

8. Kompressions-Leibteil oder Kompressionsstrumpfhose nach einem der voranstehenden Ansprüche, **dadurch gekennzeichnet, dass** sich im anterioren Bereich des Leibteils (1) distal des Bauchabschnitts (3) ein anteriorer Kompressionsabschnitt (5) aus einem Kompressionsgestrick anschließt.

9. Kompressions-Leibteil oder Kompressionsstrumpfhose nach einem der voranstehenden Ansprüche, **dadurch gekennzeichnet, dass** der posteriore Kompressionsabschnitt (2) und/oder der anteriore Kompressionsabschnitt (5) aus einem Rechts-Rechts-Flachgestrick mit darin eingebundenem oder eingelegtem Schussfaden gebildet ist.

10. Kompressions-Leibteil oder Kompressionsstrumpfhose nach Anspruch 8 oder 9, **dadurch gekennzeichnet, dass** die Kompressionsgestricke des posterioren Kompressionsabschnitts (2) und des anterioren Kompressionsabschnitts (5) miteinander verstrickt sind.

11. Kompressions-Leibteil oder Kompressionsstrumpfhose nach einem der voranstehenden Ansprüche, **dadurch gekennzeichnet, dass** der kompressionsgebende Schussfaden des Kompressionsgestricks in Umfangsrichtung verläuft.

12. Kompressions-Leibteil oder Kompressionsstrumpfhose nach einem der voranstehenden Ansprüche, **dadurch gekennzeichnet, dass** an einem proximalen Randabschnitt des Leibteils (1) ein verstellbarer Taillengurt (7) angeordnet ist.

13. Kompressions-Leibteil oder Kompressionsstrumpfhose nach Anspruch 10, **dadurch gekennzeichnet, dass** der Taillengurt (7) wenigstens einen verstellbaren Klettverschluß (6) oder wenigstens einen Verschlußknopf mit zugehörigem Knopflochband mit mehreren Knopflöchern aufweist.

14. Kompressions-Leibteil oder Kompressionsstrumpfhose nach einem der voranstehenden Ansprüche, wobei der posteriore Kompressionsabschnitt (2) bei angelegtem Leibteil (1) im Gesäß- und Lendenbereich einer Patientin zu liegen kommt.

15. Kompressions-Leibteil oder Kompressionsstrumpfhose nach einem der voranstehenden Ansprüche, wobei die Öffnungen (4) in dem Grundgestrick des Bauchabschnitts (3) ein regelmäßiges, matrixförmiges Lochmuster bilden und bevorzugt gleichförmig und regelmäßig in dem Grundgestrick des Bauchabschnitts (3) angeordnet sind.

## Claims

1. Compression body part or compression tights with body part for compression treatment of oedemas or varicosis during pregnancy, wherein the body part (1) has a posterior compression section (2) made of a compression knitted fabric with a compression-producing weft thread and an anterior abdomen section (3) which comes to rest in the abdomen region of the patient when the body part (1) is worn, **characterised in that** the abdomen section (3) is formed from a stretchable knitted fabric without weft thread having a plurality of openings (4), wherein the openings (4) in the basic knitted fabric of the abdomen section (3) are designed like a net, like a lattice or like a perforation.

2. Compression body part or compression tights according to claim 1, **characterised in that** the abdomen section (3) is formed from a right-right knitted fabric without weft thread having lattice-like or net-like openings (4).

3. Compression body part or compression tights according to claim 1 or claim 2, **characterised in that** the abdomen section (3) is formed from a knitted fabric without weft thread having a petinet hole pattern.

4. Compression body part or compression tights according to one of the preceding claims, **characterised in that** the abdomen section (3) contains an elastic flat knitted fabric made of an elastic knitting thread.

5. Compression body part or compression tights according to one of the preceding claims, **characterised in that** the knitted fabric of the abdomen section (3) is knitted to the compression knitted fabric of the posterior compression section (2) adjoining the abdomen section (3) on the edge side.

6. Compression body part or compression tights according to one of the preceding claims, **characterised in that** the openings (4) in the knitted fabric of the abdomen section (3) are knitted in by taking off stitches on their needle and putting onto their adjacent needle.

7. Compression body part or compression tights according to one of the preceding claims, **characterised in that** the compression knitted fabric of the posterior compression section (2) generates a compression pressure, which increases proximally to distally, on the seat region and lumbar region of the patient when the body part (1) is worn.

8. Compression body part or compression tights according to one of the preceding claims, **characterised in that** an anterior compression section (5) made of a compression knitted fabric is adjoined in the anterior region of the body part (1) distally of the abdomen section (3).

9. Compression body part or compression tights according to one of the preceding claims, **characterised in that** the posterior compression section (2) and/or the anterior compression section (5) is formed from a right-right flat knitted fabric with weft thread bound or inserted therein.

10. Compression body part or compression tights according to claim 8 or 9, **characterised in that** the compression knitted fabric of the posterior compression section (2) and of the anterior compression section (5) are knitted to one another.

11. Compression body part or compression tights according to one of the preceding claims, **characterised in that** the compression-producing weft thread of the compression knitted fabric runs in peripheral direction.

12. Compression body part or compression tights according to one of the preceding claims, **characterised in that** an adjustable waistband (7) is arranged on a proximal edge section of the body part (1).

13. Compression body part or compression tights according to claim 10, **characterised in that** the waistband (7) has at least one adjustable Velcro fastener (6) or at least one fastener button with associated button hole ribbon having several button holes.

14. Compression body part or compression tights according to one of the preceding claims, wherein the posterior compression section (2) comes to rest in the seat region and lumbar region of a patient when the body part (1) is worn.

15. Compression body part or compression tights according to one of the preceding claims, wherein the openings (4) in the basic knitted fabric of the abdomen section (3) form a regular, matrix-like hole pattern and are arranged preferably uniformly and regularly in the basic knitted fabric of the abdomen section (3).

## Revendications

1. Corsage de contention ou collant de contention avec un corsage pour le traitement par contention d'œdèmes ou d'une varice pendant la grossesse, dans lequel le corsage (1) présente une section de contention postérieure (2) en un tricot de contention avec un fil de trame fournissant la contention ainsi qu'une section ventrale antérieure (3), qui vient se poser, lorsque le corsage (1) est posé, dans la zone ventrale de la patiente, **caractérisé en ce que** la section ventrale (3) est formée d'un tricot extensible sans fil de trame avec une pluralité d'ouvertures (4), dans lequel les ouvertures (4) sont réalisées dans le tricot de base de la section ventrale (3) de type filet, grille ou perforation.

2. Corsage de contention ou collant de contention selon la revendication 1, **caractérisé en ce que** la section ventrale (3) est formée d'un tricot réversible sans fil de trame avec des ouvertures de type grille ou filet (4).

3. Corsage de contention ou collant de contention selon la revendication 1 ou 2, **caractérisé en ce que** la section ventrale (3) est formée d'un tricot sans fil de trame avec un motif à trou Petinet.

4. Corsage de contention ou collant de contention selon l'une quelconque des revendications précédentes, **caractérisé en ce que** la section ventrale (3) contient un tricot plat élastique en un fil de tricot élastique.

5. Corsage de contention ou collant de contention selon l'une quelconque des revendications précédentes, **caractérisé en ce que** le tricot de la section ventrale (3) est tricoté avec le tricot de contention de la section de contention postérieure (2) se raccordant côté bord à la section ventrale (3).

6. Corsage de contention ou collant de contention selon l'une quelconque des revendications précédentes, **caractérisé en ce que** les ouvertures (4) sont tricotées dans le tricot de la section ventrale (3) par retrait de mailles sur leur aiguille et suspension sur leur aiguille contiguë.

7. Corsage de contention ou collant de contention selon l'une quelconque des revendications précédentes, **caractérisé en ce que** le tricot de contention de la section de contention postérieure (2) génère, lorsque le corsage est posé (1), une pression de contention croissant du côté proximal au côté distal sur les zones des fesses et lombaire de la patiente.

8. Corsage de contention ou collant de contention selon l'une quelconque des revendications précédentes, **caractérisé en ce qu'**une section de contention antérieure (5) en un tricot de contention se raccorde, dans la zone antérieure du corsage (1), de manière distale à la section ventrale (3).

9. Corsage de contention ou collant de contention selon l'une quelconque des revendications précédentes, **caractérisé en ce que** la section de contention postérieure (2) et/ou la section de contention antérieure (5) est formée d'un tricot plat réversible avec un fil de trame intégré ou introduit dans celui-ci.

10. Corsage de contention ou collant de contention selon la revendication 8 ou 9, **caractérisé en ce que** les tricots de contention de la section de contention postérieure (2) et de la section de contention antérieure (5) sont tricotés entre eux.

11. Corsage de contention ou collant de contention selon l'une quelconque des revendications précédentes, **caractérisé en ce que** le fil de trame fournissant la contention du tricot de contention s'étend dans le sens périphérique.

12. Corsage de contention ou collant de contention selon l'une quelconque des revendications précédentes, **caractérisé en ce qu'**une ceinture réglable (7) est disposée au niveau d'une section de bord proximale du corsage (1).

13. Corsage de contention ou collant de contention selon la revendication 10, **caractérisé en ce que** la ceinture (7) présente au moins une fermeture autoagrippante réglable (6) ou au moins un bouton de fermeture avec une bande percée de bouton associée avec plusieurs trous de bouton.

14. Corsage de contention ou collant de contention selon l'une quelconque des revendications précédentes, dans lequel la section de contention postérieure (2) vient se poser, lorsque le corsage (1) est posé, dans les zones des fesses et lombaire d'une patiente.

15. Corsage de contention ou collant de contention selon l'une quelconque des revendications précédentes, dans lequel les ouvertures (4) dans le tricot de base de la section ventrale (3) forment un motif à trous régulier en forme de matrice et sont disposées de préférence uniformément et régulièrement dans le tricot de base de la section ventrale (3).
